# EUROPEAN PATENT APPLICATION

(11) **EP 3 388 049 A1**
(43) Date of publication of application: **17.10.2018**
(21) Application number: 16873195.8
(22) Date of filing: 18.08.2016
(51) Int. Cl.: A61K 8/34, A61K 8/81, A61K 8/19, A61K 8/362, A61K 8/73, A61K 8/04, A61Q 15/00, A61Q 19/10

(54) **DEODORIZING COSMETIC COMPOSITION AND METHOD FOR PREPARING SAME**

(30) Priority: 09.12.2015 KR 20150175069
(71) Applicant: Eins Co., Ltd., Seoul 06367 (KR)
(72) Inventor: LEE, Kwan-Yeop, Seoul 05378 (KR)
(74) Representative: van Dam, Vincent
(86) International application number: PCT/KR2016/009071
(87) International publication number: WO 2017/099329

(57) **Abstract**

The present invention discloses a deodorizing cosmetic composition and a method for preparing the same. This present invention constitutes a cleansing gel through a combination of antibacterial, antiviral, and deodorizing active components, wherein the cleansing gel has the properties of being free of alcohols, preservatives, toxicity, irritation, and fragrances, and can be applied to important areas of the body. Accordingly, during sexual intercourse, excellent antibacterial, antiviral, and deodorizing activities maintain hygiene in the genital area, which is an important area of the body, and effectively prevent bacterial or viral infections, such as gonorrhea or acquired immune deficiency syndrome (AIDS), during sexual intercourse between a man and a woman, regardless of the use of contraceptives. Consequently, a healthy sex life between the man and the woman can take place safely.

## Description

### TECHNICAL FIELD

The present invention relates to a cleaning gel having antibacterial and antiviral activities used by application thereof to a specific part of the body. More specifically, the present invention relates to a deodorant cosmetic composition included in the cleaning gel composition containing active ingredients having antibacterial, antiviral, and deodorizing activities and a method of preparing the deodorant cosmetic composition. The deodorant cosmetic composition has excellent antibacterial, antiviral, and deodorizing activities, so that, when the cosmetic composition is used, specific parts (e.g., a sexual organ) of the body may be kept clean, and sexually transmitted diseases, such as gonorrhea and acquired immunodeficiency syndrome (AIDS), may be effectively prevented.

### BACKGROUND ART

In general, sexuality is one of the strongest human desires, and is an affection wherein man and woman meet to confirm each other's love through physical contact.

To prevent sexually transmitted diseases such as acquired immunodeficiency syndrome (AIDS) and to avoid unwanted pregnancies during sexual intercourse between men and women, male contraceptive devices such as condoms, which are easy to use, have been used. These male contraceptive devices are recognized as necessities of life, and thus can be easily purchased at drug stores, convenience stores, and vending machines in public places.

A male contraceptive device is manufactured by molding a rubber having excellent elasticity and liquid barrier properties into an approximately cylindrical shape so as to prevent semen from leaking to the outside or to prevent foreign substances from penetrating into the contraceptive device. In addition, the contraceptive device is made as thin as possible to prevent deterioration of sexual feeling.

Meanwhile, the male contraceptive device made of rubber has a large coefficient of friction. Accordingly, to reduce friction generated when using the contraceptive device, a lubricant such as a gel is applied to the surface of the contraceptive device or an auxiliary agent capable of imparting chemical stimulus to a male or female body is used. Therefore, when the male contraceptive device is used at the time of sexual intercourse, it is possible to prevent semen from entering into the uterus, thereby preventing unwanted pregnancy, and prevent male genitalia from being exposed to foreign substances, thereby preventing sexually transmitted diseases, such as gonorrhea and AIDS.

However, the male contraceptive device has the disadvantage of being easily torn. Also, users tend to avoid wearing the male contraceptive device. As a result, sexually transmitted diseases are often not prevented properly. Therefore, special measures are needed to prevent sexually transmitted diseases.

Meanwhile, various cleansing agents having an antibacterial or antiviral function capable of removing bacteria and the like present on the hands and feet are commercially available. However, when these cleansing agents were applied to the genitalia, sexually transmitted disease prevention effect was not observed.

### DISCLOSURE

### TECHNICAL PROBLEM

Therefore, the present invention has been made in view of the above problems, and it is one object of the present invention to provide a deodorant cosmetic composition containing active ingredients having antibacterial, antiviral, and deodorizing activities and a method of preparing the same, wherein the deodorant cosmetic composition has non-alcoholic, non-preservative, non-toxic, non-irritating, and odorless properties, and is contained in a cleaning gel composition that may be used by application thereof to a specific part of the body. Since the deodorant cosmetic composition has excellent antibacterial, antiviral, and deodorizing activities, use of the cleaning gel composition containing the deodorant cosmetic composition at the time of sexual intercourse may maintain cleanliness of a sexual organ, which is an important part of the body, and may effectively prevent infection with bacteria or viruses without use of a contraceptive device, thereby preventing sexually transmitted diseases, such as gonorrhea and acquired immunodeficiency syndrome (AIDS). Ultimately, the deodorant cosmetic composition of the present invention enables healthy sexual life of men and women.

### TECHNICAL SOLUTION

In accordance with one aspect of the present invention, provided is a deodorant cosmetic composition, wherein the deodorant cosmetic composition is prepared by mixing 12 to 18 wt% of butylene glycol, 7 to 13 wt% of glycerin, and 1.11 to 5.85 wt% of additives in 65 to 79.89 wt% of purified water (AQUA Inc.), wherein the additives include sodium polyacrylate, a catalyst, a carbomer, an inorganic carrier, a metal salt, a silver precursor, citric acid, and hydroxyethylcellulose.

In addition, the additives may be obtained by mixing 1 to 4 wt% of sodium polyacrylate, 0.01 to 0.30 wt% of the catalyst, 0.01 to 0.30 wt% of the carbomer, 0.01 to 0.30 wt% of the inorganic carrier, 0.01 to 0.30 wt% of the metal salt, 0.05 to 0.30 wt% of the silver precursor, 0.01 to 0.05 wt% of citric acid, and 0.01 to 0.30 wt% of hydroxyethylcellulose.

In addition, the catalyst may be at least one selected from the group consisting of aqueous ammonia, ammonium bicarbonate, and triethanolamine.

In addition, the inorganic carrier may be at least one selected from the group consisting of silica, zeolite, and zirconium, which have an average particle diameter of 100 to 150 nm.

In addition, the metal salt may be at least one selected from the group consisting of metal nitrates, metal phosphates, and metal carbonates.

In addition, the silver precursor may be at least one selected from the group consisting of silver nitrate, silver nitrite, and silver perchlorate.

In accordance with another aspect of the present invention, provided is a method of preparing a deodorant cosmetic composition including (a) a step, in which first mixing is performed by mixing 12 to 18 wt% of butylene glycol with 65 to 79.89 wt% of purified water (AQUA Inc.); (b) a step, in which secondary mixing is performed by mixing the first mixture obtained in step (a) with additives including 1 to 4 wt% of sodium polyacrylate, 0.01 to 0.30 wt% of a catalyst, 0.01 to 0.30 wt% of a carbomer, 0.01 to 0.30 wt% of an inorganic carrier, 0.01 to 0.30 wt% of a metal salt, 0.05 to 0.30 wt% of a silver precursor, 0.01 to 0.05 wt% of citric acid, and 0.01 to 0.30 wt% of hydroxyethylcellulose, wherein the additives contain active ingredients having antibacterial, antiviral, and deodorizing activities, and then a reducing agent is added to the secondary mixture to form a bonding layer on the surface of the inorganic carrier, wherein the bonding layer is formed by reacting the metal salt and the silver precursor in the presence of the reducing agent at a temperature range of 35 to 38 °C for 3 to 4 hours; and, (c) a step, in which secondary mixing is performed by mixing the secondary mixture obtained in step (b) with 7 to 13 wt% of glycerin as a dispersion solvent and the mixture is dispersed at a speed of 100 to 500 rpm, and a cleaning gel composition, which has antibacterial, antiviral, and deodorizing activities and is used by application thereof to a specific part of the body, is prepared.

In addition, the reducing agent may be ascorbic acid or sodium borohydride and may be used in an amount of 0.1 to 0.5 parts by weight based on 100 parts by weight of the cleaning gel composition.

### ADVANTAGEOUS EFFECTS

As apparent from the foregoing, the present invention advantageously provides a deodorant cosmetic composition containing active ingredients having antibacterial, antiviral, and deodorizing activities, wherein the deodorant cosmetic composition has non-alcoholic, non-preservative, non-toxic, non-irritating, and odorless properties, and is contained in a cleaning gel composition that can be used by application thereof to a specific part of the body. Since the deodorant cosmetic composition has excellent antibacterial, antiviral, and deodorizing activities, use of the cleaning gel composition containing the deodorant cosmetic composition at the time of sexual intercourse can maintain cleanliness of a sexual organ, which is an important part of the body, and can effectively prevent infection with bacteria or viruses without use of a contraceptive device, thereby preventing sexually transmitted diseases, such as gonorrhea and acquired immunodeficiency syndrome (AIDS). Ultimately, the deodorant cosmetic composition of the present invention enables healthy sexual life of men and women.

### DESCRIPTION OF DRAWINGS

FIGS. 1 and 2 include graphs showing results obtained in skin irritation tests performed in examples of the present invention, showing viability over time.
FIG. 3 is a graph showing inhibitory concentrations for HIV-1 in an example of the present invention. The graph was created using GraphPad Prism.
FIG. 4 is a graph showing viability of C8166 cells depending on the concentrations of the test product of an example of the present invention. The graph was created using GraphPad Prism.

### BEST MODE

The present invention will now be described more fully with reference to the accompanying drawings, in which exemplary embodiments of the invention are shown. The invention may, however, be embodied in many different forms and should not be construed as being limited to the embodiments set forth herein; rather, these embodiments are provided so that this disclosure will be thorough and complete, and will fully convey the concept of the invention to those skilled in the art. The present disclosure is defined only by the categories of the claims.

The terms used in the present specification are used to explain a specific exemplary embodiment and not to limit the present inventive concept. Thus, the expression of singularity in the present specification includes the expression of plurality unless clearly specified otherwise in context.

In the present specification, terms such as "include" or "comprise" should be construed as denoting that a certain characteristic, number, step, operation, constituent element, component or a combination thereof exists and not as excluding the existence of or a possibility of an addition of one or more other characteristics, numbers, steps, operations, constituent elements, components or combinations thereof.

In addition, embodiments described herein will be described with reference to cross-sectional views and/or plan views. In the drawings, the thicknesses of the films and regions are exaggerated for an effective description of the technical content. Thus, the shape of the illustrations may be modified by manufacturing techniques and/or tolerances. Accordingly, the embodiments of the present invention are not limited to the specific forms, but may include variations in shapes that are created or required according to the manufacturing process. For example, the area shown at right angles may be rounded or may have a shape with a certain curvature. Accordingly, the regions illustrated in the drawings have schematic attributes, and the shapes of the regions illustrated in the drawings are intended to illustrate specific types of regions of the apparatus and are not intended to limit the scope of the invention.

Like reference numerals designate like elements throughout the specification. Therefore, although the same reference numerals or similar reference numerals are not mentioned or described in the drawings, they may be described with reference to other drawings. In addition, although the reference numerals are not shown, they can be described with reference to other drawings.

Hereinafter, embodiments of the present invention will be described.

A method of preparing the deodorant cosmetic composition according to an embodiment of the present invention includes steps (a), (b), and (c).

In step (a), first mixing is performed by mixing 65 to 79.89 wt% of purified water with 12 to 18 wt% of butylene glycol as an alcohol solvent having antimicrobial activity.

In step (b), secondary mixing is performed by mixing the first mixture obtained in step (a) with additives including 1 to 4 wt% of sodium polyacrylate, 0.01 to 0.30 wt% of a catalyst, 0.01 to 0.30 wt% of a carbomer, 0.01 to 0.30 wt% of an inorganic carrier, 0.01 to 0.30 wt% of a metal salt, 0.05 to 0.30 wt% of a silver precursor, 0.01 to 0.05 wt% of citric acid, and 0.01 to 0.30 wt% of hydroxyethylcellulose, wherein the additives contain active ingredients having antibacterial, antiviral, and deodorizing activities, and then a reducing agent is added to the secondary mixture to form a bonding layer on the surface of the inorganic carrier, wherein the bonding layer is formed by reacting the metal salt and the silver precursor in the presence of the reducing agent at a temperature range of 35 to 38 °C for 3 to 4 hours.

In this case, sodium polyacrylate is a sodium salt of an acrylic acid polymer and a water-soluble thickener. Sodium polyacrylate exists in a coil state, but when it comes into contact with water (H2O), the COOH group of sodium polyacrylate dissociates into COO- while being switched to an uncoiled state, maximizing ion repulsion and forming viscosity. Through such a mechanism, when the cleaning gel composition prepared according to an embodiment of the present invention is applied to the skin, a coating film may be formed.

The catalyst may be at least one selected from the group consisting of aqueous ammonia, ammonium bicarbonate, and triethanolamine, preferably triethanolamine, without being limited thereto.

The carbomer as a thickener is an acidic polymer compound mainly obtained by polymerizing acrylic acid, and serves to increase the viscosity of the cleaning gel composition prepared according to an embodiment of the present invention.

The inorganic carrier may be at least one selected from the group consisting of silica, zeolite, and zirconium, which have an average particle diameter of 100 to 150 nm, preferably silica, without being limited thereto.

In this case, the silica may be prepared by stirring a silicon alkoxide in an alcohol solvent at 35 to 38 °C for 6 to 7 hours in the presence of the catalyst, wherein the silica is prepared in a particle form having an average particle size of 100 to 150 nm. The silica particles prepared in this manner are capable of functioning as an antimicrobial and antiviral carrier, a deodorizing ingredient, and a far-infrared emitter.

In this case, in terms of antibacterial and antiviral activities and stability, it is important to distribute the particle diameter within a certain range. When the particle diameter of the particles is less than 100 nm, there is a possibility of the nanopowder penetrating the human body through the skin or the like to cause toxicity. On the other hand, when the particle diameter of the particles exceeds 150 nm, the surface area of the particles may decrease, resulting in deterioration in antibacterial and antiviral properties.

In addition, the alcohol solvent includes methanol, ethanol, propanol, and the like, and the content thereof is 700 to 800 parts by weight based on 100 parts by weight of the silicon alkoxide. When the content is out of the above range, the viscosity of the reaction liquid is too large or too small, which is not preferable.

In addition, the reaction time may be 6 to 7 hours. When the reaction time is less than 6 hours, the reaction does not occur sufficiently. On the other hand, when the reaction time exceeds 7 hours, uneven distribution of the silica powder particle size due to generation of secondary particles after generation of primary particles may be caused, which is not preferable. The reaction temperature may be 35 °C to 38 °C. When the reaction temperature is less than 35 °C, the reaction time becomes longer. On the other hand, when the reaction temperature exceeds 38 °C, the particle size may not be uniform.

In addition, the silica particles obtained in the process are mixed with a silver precursor and any one metal salt selected from the group consisting of zinc (Zn), magnesium (Mg), calcium (Ca), and copper (Cu) in an alcohol solvent. Then, a reducing agent is added to the mixture to reduce the silver precursor, so that the reduced silver precursor and the metal salt form a bonding layer having antibacterial and antiviral activities and a deodorizing component on the surfaces of the silica particles.

The process of forming the bonding layer having antibacterial and antiviral activities and a deodorizing component may be performed in an alcohol solvent at 35 to 38 °C for 3 to 4 hours so that the silver precursor is reduced and the metal salt is sufficiently bonded to the surfaces of the silica particles. When the reaction time is less than 3 hours, the reaction may not proceed sufficiently and thus bonds may not be sufficiently formed. When the reaction time exceeds 4 hours, economic efficiency is lowered, which is not preferable.

The silver precursor may include silver nitrate, silver nitrite, silver perchlorate, and the like, and the metal salt may include metal nitrates, phosphates, and carbonates.

The total amount of the silver precursor and the metal salt mixed in the alcohol solvent is preferably 0.15 to 0.50 wt% based on 100 wt% of the cleaning gel composition prepared according to an embodiment of the present invention. When the amount is less than 0.15 wt%, the bonding layer may not be sufficiently formed, and thus antibacterial, antiviral, and deodorizing activities may be reduced. When the amount is more than 0.50 wt%, too much bonding is formed in the silica particles, thereby deteriorating far-infrared emitting efficiency and increasing the number of unreacted substances, which may reduce economic efficiency.

When the silver precursor and the metal salt are mixed, the silver precursor is most preferably contained in an amount of 0.05 to 0.20 wt% and the metal salt is most preferably contained in an amount of 0.1 to 0.3 wt%.

The alcohol solvent used in formation of the bonding layer may include methanol, ethanol, propanol, and a mixture thereof, and may be used in an amount of 2,550 to 2,650 parts by weight based on 100 parts by weight of the silica particles.

Conventional reducing agents, such as ascorbic acid and sodium borohydride (NaBH4), may be used as the reducing agent used to reduce the silver precursor at the time of formation of the bonding layer. The reducing agent may be contained in an amount of 0.1 to 0.5 parts by weight based on 100 parts by weight of the silica particles.

After the process of forming the bonding layer having antibacterial and antiviral activities and a deodorizing component is completed, the product is preferably subjected to washing, drying and heat treatment processes according to an appropriate method to remove impurities and unreacted materials.

Through the process of forming the bonding layer having antibacterial and antiviral activities and a deodorizing component, a bonding layer containing silver (Ag) and metal particles and having a thickness of 2 to 5 nm is uniformly formed on the surfaces of the silica particles.

When the bonding layer having antibacterial and antiviral activities and a deodorizing component is formed, the silver and the metal particles are preferably uniformly bonded in the form of agglomerated particles on the surfaces of the silica particles.

When the bonding layer covers the entire surface of the silica particle, the far-infrared emitting efficiency of the silica particles is lowered, which is undesirable.

Silver precursors, such as silver nitrate, silver nitrite, and silver perchlorate, are expensive, and thus use of only silver precursors is not economic. In an embodiment of the present invention, a relatively inexpensive metal salt, such as zinc, magnesium, calcium, and copper, is used together with silver rather than using only the expensive silver precursor. Nevertheless, a deodorant cosmetic composition having excellent activity may be prepared at low cost with a simple process.

In addition to antibacterial, antiviral, and deodorizing activities, the deodorant cosmetic composition prepared according to an embodiment of the present invention has antifungal activity and an air purification function according to far-infrared emitting activity.

According to a preferred embodiment of the present invention, the cleaning gel composition is dispersed in a dispersion medium, preferably any one selected from the group consisting of alcohols, water, 1,3-butylene glycol, glycerin, and polyethylene glycol, more preferably glycerin, using a high-speed Aji mixer. In this case, the speed of the mixer is preferably adjusted to 100 to 500 rpm. When the speed is less than 100 rpm, dispersion may not be performed well, and thus precipitation and discoloration may occur after preparation of an aqueous composition. When the speed exceeds 500 rpm, energy consumption increases.

The cleaning gel composition according to an embodiment of the present invention may further include a fragrance. Since the cleaning gel composition according to an embodiment of the present invention includes porous silica, the composition exhibits a deodorizing effect through inhibition of odor causing bacteria by antibacterial components. In addition, an excellent deodorizing effect may be achieved by masking odor using a fragrance.

In an embodiment of the present invention, a long-lasting fragrance is preferable, and a long-lasting fragrance having increased content of base note ingredients compared to top note ingredients is more preferable.

According to a preferred embodiment of the present invention, the fragrance may be used in an amount of 0.01 to 0.10 wt% based on the total weight of the composition, without being limited thereto.

When a hydrophobic fragrance is used, ethanol and a surfactant may be used together to uniformly distribute the fragrance in the cleaning gel composition and maintain transparency. In this case, ethanol may be used in an amount of 5 to 20 wt%, preferably 10 to 15 wt%, based on the total weight of the composition. The amount of the surfactant used is 1 to 4 times or more the amount of the fragrance used, preferably 0.1 to 0.5 wt%. The preferred proportions of fragrance, ethanol, and the surfactant should be determined within a range that achieves a uniform distribution and transparency of the composition. As the surfactant, a commonly-used cationic, anionic, or nonionic surfactant may be used, preferably an anionic or nonionic surfactant, more preferably an anionic surfactant.

When the cleaning gel composition according to an embodiment of the present invention is prepared, a concentration control agent, preferably citric acid, may be used. The cleaning gel composition according to an embodiment of the present invention prepared by the above method may be formulated into a preparation that may be applied to a genital area, which is an important part of a male, according to a conventional method.

### EXAMPLES

Hereinafter, skin safety test and antibacterial and HIV tests for the deodorant cosmetic composition according to an embodiment of the present invention performed at the Life Science Institute (BioScience Laboratories, Inc.) will be described.

Address and contact information for the Life Science Institute (BioScience Laboratories, Inc.) are as follows. Address: 1775 South 19th Avenue Bozeman, MT 59718, Telephone number: 406-587-5735, Fax number: 406-586-7930, Website address: www.biosciencelabs.com

### [1] Skin safety test

The skin inflammation potential of a cleaning gel of product No. 1 (also known as Double. S Gel (Lot No. 15730A2); hereinafter, referred to as experimental product)) was evaluated using commercially available MTT effective time (ET-50) live cell experiment test and a skin tissue model.

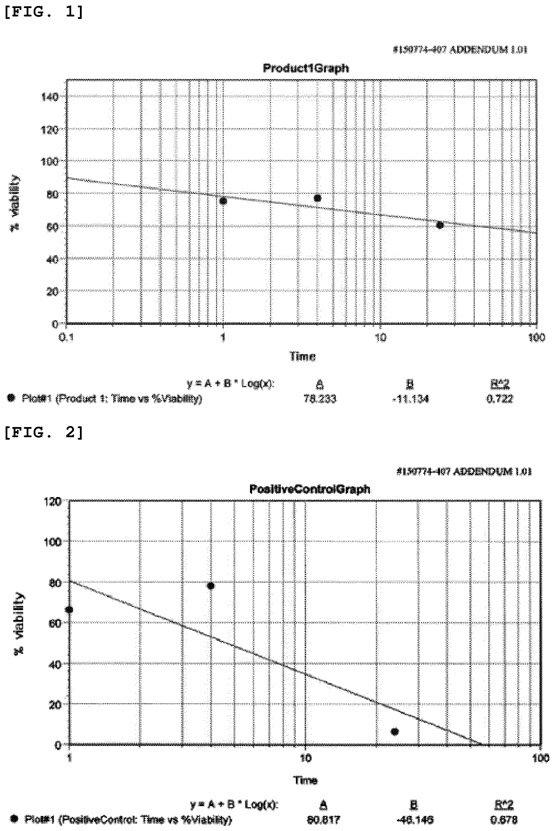

The experiments performed at the Life Science Institute (BioScience Laboratories, Inc.) began on September 2, 2015 and were completed on September 4, 2015. One deviation in SOP generated during the experiments is shown in Tables 1 to 7 below, the protocols and/or SOP deviation recording formats (form number: 99-QA-004) of FIGS. 1 and 2. The deviation did not affect the study results. The skin tissue samples were exposed to the experimental product for 1 hour, 4 hours, or 24 hours.

The experimental product was tested undiluted, as provided by the sponsor. Three untreated negative control dermal tissue specimens were exposed for 24 hours.

The first tissue specimen, the second tissue specimen, and the third tissue specimen were exposed to 1.0 % Triton X-100 (Lot No. 082615BBA) as a positive control for 1, 4, and 24 hours, respectively.

At the same time as the experimental procedure, MTT control was evaluated, and the evaluation was regarded as a result of direct reduction of the MTT product. The experimental product directly reduced MTT. During the experimental procedure, the frozen tissue was exposed to the experimental product to determine whether the experimental product was effectively washed from tissue.

The results obtained from the frozen tissue indicate that the experimental product may be washed out of the tissue, indicating that the results are valid.

The absorbance of the specimens was determined using a VERSA maxTM Tunable Microplate Reader (with SOFTmaxⓡ PRO Software) set to 570 nm with several 200 pm aliquots of isopropyl alcohol.

Absorbance values were converted into the probabilistic feasibility of tissue culture by SOFTmaxⓡ PRO Software. These values were expressed as time-dependent by SOFTmaxⓡ PRO Software, and then ET-50 was determined by interpolation. ET-50 is an estimated time when the probabilistic feasibility of tissues falls to 50%.

### (1) Experimental Example 1 of skin safety test

MTT ET-50 in hours-Product 1 = 343.39
MTT ET-50 in hours-Positive Control = 4.65
Expected In-Vivo Irritancy-Product 1-Non Irritating
Expected In-Vivo Irritancy-Positive Control-Moderate to Mild Irritation
Experimental product (Product #1) = Cleaning gel (Double. S Gel (Lot #15730A2))
Positive Control = 1% Triton X-100 solution (Lot #082615BBA)
Negative Control = Non-administered tissue
Str./Sev. Irrit. = Strong/Severe Irritation (Possibly Corrosive)
Mod. Irrit. = Moderate Irritation
Mod. to Mild Irrit. = Moderate to Mild Irritation
Very Mild Irrit. = Very Mild Irritation
Non-Irrit. = Non-Irritation

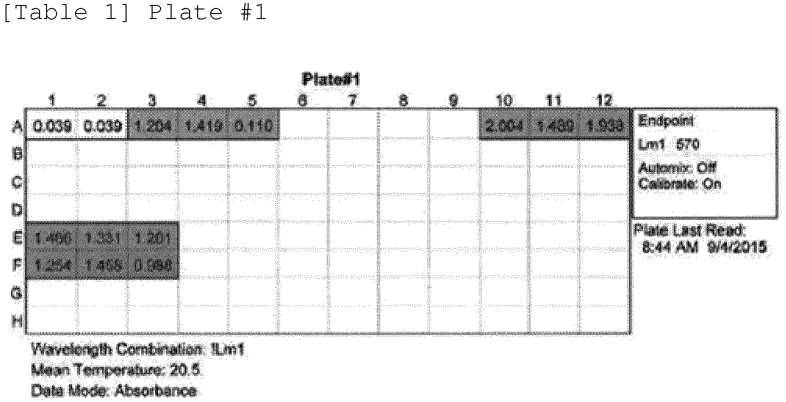

**[Table 2] Product 1 (Hours)**

| **Product 1 (Hours)** | | | | | | |
|---|---|---|---|---|---|---|
| Sample | Wells | Time | Values | MeanValue | %Viability | CV% |
| 01 | E1 | 1 | 1.466 | 1.360 | 75.13 | 11.01 |
| | F1 | | 1.254 | | | |
| 02 | E2 | 4 | 1.331 | 1.394 | 77.03 | 6.40 |
| | F2 | | 1.458 | | | |
| 03 | E3 | 24 | 1.201 | 1.095 | 60.47 | 13.76 |
| | F3 | | 0.988 | | | |

**[Table 3] Positive Control (Hours)**

| **PositiveControl (Hours)** | | | | |
|---|---|---|---|---|
| Sample | Wells | Time | Values | %Viability |
| 01 | A3 | 1 | 1.204 | 66.51 |
| 02 | A4 | 4 | 1.419 | 78.40 |
| 03 | A5 | 24 | 0.110 | 6.06 |

**[Table 4] Negative Control (Hours)**

| **NegativeControl (Hours)** | | | |
|---|---|---|---|
| Sample | Wells | Time | Value |
| 01 | A10 | 24 | 2.004 |
| 02 | A11 | 24 | 1.489 |
| 03 | A12 | 24 | 1.938 |
| Negative Control Value = 1.810 | | | |

### (2) Experimental Example 2 of Killed Tissue Control Test

Product #1 = Double. S Gel (Lot #15730A2)
Negative Control = Non-administered tissue

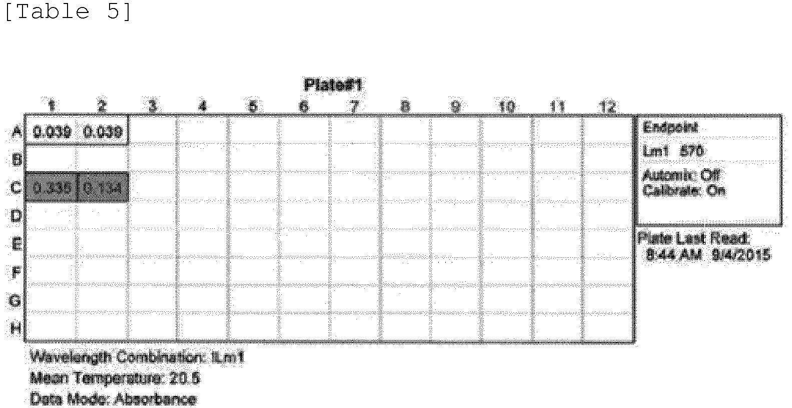

**[Table 6]**

| **Product1 (Hours)** | | | |
|---|---|---|---|
| Sample | Wells | Time | Value |
| 01 | C2 | 24 | 0.134 |

**[Table 7]**

| **NegativeControl (Hours)** | | | |
|---|---|---|---|
| Sample | Well | Time | Value |
| 01 | C1 | 24 | 0.335 |
| Negative Control Value = 0.335 | | | |

### (3) Skin irritation test results

ET-50 of Test product #1 was determined. ET-50 of the experimental product was 343.39 hours under the condition of non-irritating. This was confirmed from the results of the skin irritation test on the experimental product shown in Table 8 below.

**[Table 8]**

| **Dermal Irritation Results; MTT Effective Time-50 (ET-50) Viable Cell Assay Test** | | | |
|---|---|---|---|
| Product # | Product Description | MTT ET-50 (in hours) | Irritancy Classification |
| 1 | Double. S Gel (Lot #15730A2) | 343.309 | Non-Irritating |
| Positive Control | 1% Triton X-100 Solution (Lot #082615BBA) | 4.65 | Moderate to Mild Irritation |

### [2] Antibacterial test

Experiments on a study (# 150773-403) on "GLP Assessment of Human Immunodeficiency Virus Type-1 (HIV-1) versus One Compounding Agent for Antiviral Activity" were completed. The experiments started on August 24, 2015 and were completed on October 12, 2015.

One experimental product was provided for evaluation. Antiviral responses to human immunodeficiency virus (HIV-1, cell line Mn; ZeptoMetrix, Corp. #0810027CF) were assessed in the experimental product diluted in medium at eight different concentrations. Cytotoxic and antiviral experiments were repeated twice for each test. All experiments were performed according to Good Laboratory Practices described in 40 CFR 160, except that characterization of the identity, strength, purity, composition, stability, and solubility of the experimental product was under the responsibility of the research sponsor and was not performed by the laboratory (GLP 58.105). One deviation from the study protocol occurred during the evaluation process. The volumes of the experimental product and the virus to be evaluated were changed. The difference was intentional and did not have any negative effect on the results of the study. No deviations were found from applicable BSLI Standard Operating Procedures.

The experimental product was provided to the laboratory by a research sponsor. Responsibility for determination of the identity, strength, purity, composition, stability, and solubility of the experimental product as well as preservation of the experimental product was left to the sponsor. The experimental product will be described as follows.
(1) The concentration of the experimental product was adjusted using cell culture medium.
   Experimental product #1: Cleaning gel (Double. S Gel Double S. Gel)
   Lot number: 15730A2
   Date of manufacture: July 30, 2015
   Expiration date: July 30, 2018 (3 years)
   Antiviral cell line: H1V-1 cell line Mn; (ZeptoMetrix, Corp. #0810027CF)
   Virus capacity: 0.1 MOI (Multiplicity of Infection)
   Host cells: C8166 (Human T cell leukemia [ECACC #88051601])
   Storage medium: RPMI-1640 containing 2% fetal serum and anti- (penicillin-streptomycin-amphotericin B)
(2) Methodology

Antiviral properties and cytotoxicity were analyzed using cytopathic effect (CPE)-based assay. Cytopathic effect (CPE) was determined using MTT cell proliferation assay. For evaluation of antiviral properties, the experimental product diluted in medium at 8 different concentrations was used. Cytopathic effect (CPE) was determined using 3-(4,5-dimethylthiazolyl-2)-2,5-diphenyltetrazolium bromide (MTT) cell proliferation assay and plaque assay. Inhibitory concentration of the experimental product (IC5o and/or IC90) was determined. IC values were expressed as percent concentration and calculated using nonlinear regression analysis (GraphPad Prism 5.0 software).

The antiviral experiment was performed as follows.

Experiments: PBS was added to host cell culture and centrifugation was performed at 1500 rpm for 10 minutes. After centrifugation, PBS was discarded and 2.0 mL of the concentrated product was added to the cell pellet, and 100 µL of the cell/product mixture was added to each well of a 96-well plate in six replicates at each concentration. The plates were incubated in a 37 °C CO2 incubator for 1 hour. After incubation, 100 µL of 0.1 MOI virus was added to the cell/product mixture (100 µL medium in cytotoxic control), and the plates were placed in the incubator. After completion of culture, CPE cultures were evaluated using MTT assay.

10 µL of a MTT reagent was added to each well of the plate, and the plate was placed in the incubator, followed by incubation at 37±2 °C for at least 3 hours. After incubation, culture medium was discarded and 100 µL of DMSO was added to dissolve 100µL formazan. The absorbance of the samples was measured using a VERSAmaxⓡ Tunable Microplate Reader (using SOFTmaxⓡ PRO Software). At this time, blank was set to 570 nm.

The results were calculated as percent CPE inhibition, wherein 100 % CPE inhibition was approximately equal to the mean value of the cell control.

Results: Inhibitory and cytotoxic concentrations for HIV-1 cell line Mn are shown in Table 9.

**[Table 9] Summary of inhibitory concentrations for HIV-1 and toxic concentrations for C8166 cells**

| Test Product Designation | IC50, % | | IC90, % | | Cytotoxicity, IC50 % | | Therapeutic Index (TC50/IC50) |
|---|---|---|---|---|---|---|---|
| | Best Fit Value | 95% CI | Best Fit Value | 95% CI | Best Fit Value | 95% CI | |
| Double S Gel Lot # 15730A2 | 0.138 | 0.109 to 0.174 | 1.343 | 0.778 to 2.319 | 2.049 | 1.627 to 2.579 | 14.85 |

| | | | | | | | |
|---|---|---|---|---|---|---|---|
| IC50 = Inhibitory Concentration 50%; IC90 = Inhibitory Concentration 90%; TC50 = Toxic Concentration 50%; CI = Coefficient of Variation. | | | | | | | |

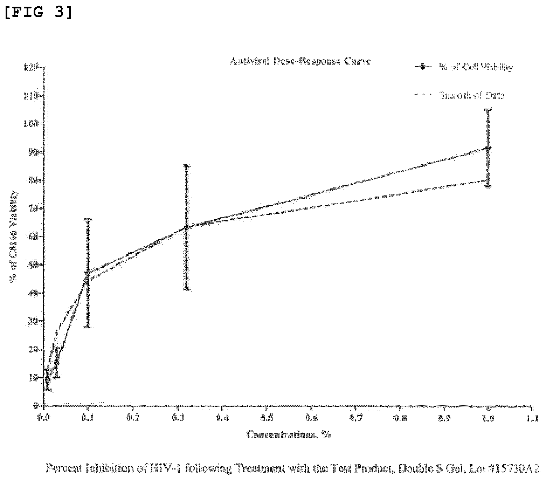

In addition, inhibition concentrations for HIV-1 calculated using a graph pad prism are shown in Tables 10 and 11 below and FIG. 3.

**[Table 10]**

| Test Product, Double S Gel, Lot #15730A2 | |
|---|---|
| Inhibitory Concentration 50% (IC50) for HIV-1 | |
| [GraphPad Prism 5.0 log (inhibitor) vs response -- Variable slope (four parameters)] | |
| log(inhibitor) vs. response-Variable slope (four parameters) | Results |
| Best-fit values | |
| Bottom | -0.0 |
| Top | -100.0 |
| LogIC50 | -0.8617 |
| HillSlope | 0.9640 |
| IC50 | 0.1375 |
| Span | -100.0 |

| Sid Error | |
|---|---|
| LogIC50 | 0.05080 |
| HillSlope | 0.1031 |

| 95% Confidence Intervals | |
|---|---|
| LogIC50 | -0.9634 to -0.7600 |
| HillSlope | 0.7576 to 1.170 |
| IC50 | 0.1088 to 0.1738 |

| Goodness of Fit | |
|---|---|
| Degrees of Freedom | 58 |
| R square | 0.8101 |
| Absolute Sum of Squares | 12853 |
| Sy.x | 14.89 |

| Constraints | |
|---|---|
| Bottom | Bottom = 0.0 |
| Top | Top = 100.0 |

**[Table 11]**

| Test Product, Double S Gel, Lot #15730A2 | |
|---|---|
| Inhibitory Concentration 90% (IC90) for HIV-1 | |
| (GraphPad Prism 5.0) | |
| log(agonist) vs. response -- Find ECanything | Results |
| Best-fit values | |
| logECF | 0.1282 |
| HillSlope | 0.9640 |
| F | - 90.00 |
| Bottom | - 0.0 |
| Top | - 100.0 |
| ECF | 1.343 |
| Span | - 100.0 |

| Sid. Error | |
|---|---|
| logECF | 0.1184 |
| HillSlope | 0.1031 |

| 95% Confidence Intervals | |
|---|---|
| logECF | -0.1089 to 0.3652 |
| HillSlope | 0.7576 to 1.170 |
| ECF | 0.7782 to 2.319 |

| Goodness of Fit | |
|---|---|
| Degrees of Freedom | 58 |
| R square | 0.8101 |
| Absolute Sum of Squares | 12853 |
| Sy.x | 14.89 |

| Constraints | |
|---|---|
| F | F = 90.00 |
| Bottom | Bottom = 0.0 |
| Top | Top = 100.0 |

| Number of points | |
|---|---|
| Analyzed | 60 |

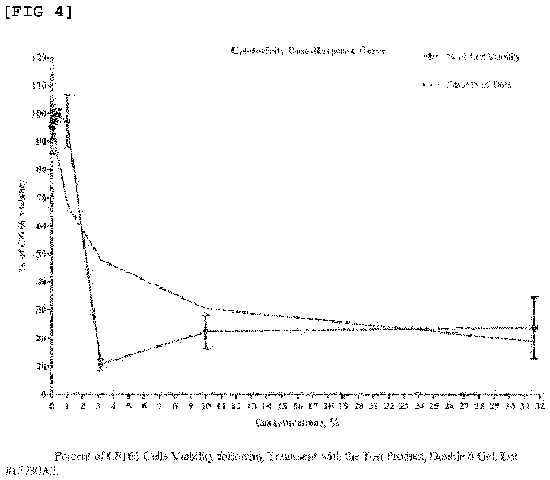

In addition, concentrations of C8166 cells calculated using a GraphPad Prism are shown in Table 12 below and FIG. 4.

**[Table 12]**

| Test Product, Double S Gel. Lot #15730A2 | |
|---|---|
| Toxic Concentration 50% (TC50) for C8166 cells | |
| [GraphPad Prism 5.0 log (inhibitor) vs response - Variable slope (four parameters)] | |
| log(inhibitor) vs. response - Variable slope (four parameters) | Results |
| Best-fit values | |
| Bottom | = 0.0 |
| Top | = 100.0 |
| LogIC50 | 0.3115 |
| HillSlope | -4.806 |
| IC50 | 2.049 |
| Span | = 100.0 |

| Sid Error | |
|---|---|
| LogIC50 | 0.05030 |
| HillSlope | 1.191 |
| 95% Confidence Intervals | |
| LogIC50 | 0.2115 to 0.4115 |
| HillSlope | -7.174 to -2.438 |
| IC50 | 1.627 to 2.579 |

| Goodness of Fit | |
|---|---|
| Degrees of Freedom | 94 |
| R square | 0.8796 |
| Absolute Sum of Squares | 17409 |
| Sy.x | 13.61 |

| Constraints | |
|---|---|
| Bottom | Bottom = 0.0 |
| Top | Top = 100.0 |

### [3] HIV prevention test

### (1) Title: GLP assessment for human immunodeficiency virus type 1 (HIV-1) versus one compounding agent for virucidal activity

Sponsor: EINS CORPORATION
Hosan bldg. 3f #222 Dosan-daero Gangnam-Gu, Seoul, Korea
Experiment institute: BIOSCIENCE LABORATORIES, INC. 1755 South 19th Avenue Bozeman, Montana 59718
Research director: Volha Dzyakanava, Ph.D.
Object: This study was conducted to evaluate the virucidal properties of one experimental product in an immunity test for human immunodeficiency virus type-1 (HIV-1) using a virucidal suspension experiment (In-Vitro Time-Kill method; standard assay for analysis of bactericidal activity against viruses in suspension) according to ASTM E1052-11. All experiments were performed according to Good Laboratory Practices described in 40 CFR 160, except that characterization of the identity, strength, purity, composition, stability, and solubility of the experimental product was under the responsibility of the research sponsor and was not performed by the laboratory (GLP 58.105).
Scope: In this study, using a virucidal suspension experiment (In-Vitro Time-Kill method), the virucidal properties of one experimental product in an immunity test for HIV-1 cell line Mn were evaluated. The experimental product was evaluated at a final concentration of 90 % (v/v). Plating was repeated four times. Virus titers were determined using a 50% tissue culture infectious dose (TCID50) quantitative method - Quantal test (Spearman-Karber Method). Percent and log10 reduction from the original population were determined after 30 and 60 second exposure of the virus cell line to the experimental product.

Research protocol explains research methodology as presented in General Data Gathering Form (Form No. 91-L-002) in Addendum 3. One deviation from the methodology described in the study protocol occurred during the evaluation process. Protocol and/or SOP Deviation Recording Form (Form No. 99-QA-004). No deviation from an applicable Standard Operating Procedure occurred during the evaluation.

### (2) Study dates

Research start date: 08/24/2015
Experiment start date: 09/09/2015
Experiment end date: 09/16/2015
Research completion date: 10/07/2015

### (3) Experimental materials

The evaluated experimental product was provided to the experiment institute by the research sponsor. Responsibility for determination of the identity, strength, purity, composition, stability, and solubility of the experimental product as well as preservation of the experimental product was left to the research sponsor.
Experimental product #1: Cleaning gel (Double. S Gellot No. 15730A2)
Date of manufacture: July 30, 2015
Expiration date: July 30, 2018 (3 years)
Antiviral cell line: Human immunodeficiency virus (HIV-1, cell line Mn; ZeptoMetrix, Corp. #0810027CF)
Host cells: C8166 (Human T cell leukemia [ECACC #88051601])
Articles and equipment: The articles and equipment used in the antibacterial test of [2] were used.
Medium: The medium used in the antibacterial test of [2] were used.

Host cell preparation: C8166 cells stored in a suspension in disposable cell culture experiment labware were used for HIV-1 experiments. Growth medium (GM) was replaced with storage medium (MM). The number of cells used in the experiment was about 4 105 cells/mL.

Experimental virus preparation: The experimental virus used in this study was prepared from a BSLI high titer virus stock. On the day of use, a certain fraction of the stock was taken out of a refrigerator at -70 °C and thawed before use in the experiment.

Table 13 below provides data from virus infectivity control (TCID50) and post-exposure infectivity (TCID50). LOG10 and percent reduction were observed after 30 and 60 second exposure of HIV-1 to the test product.

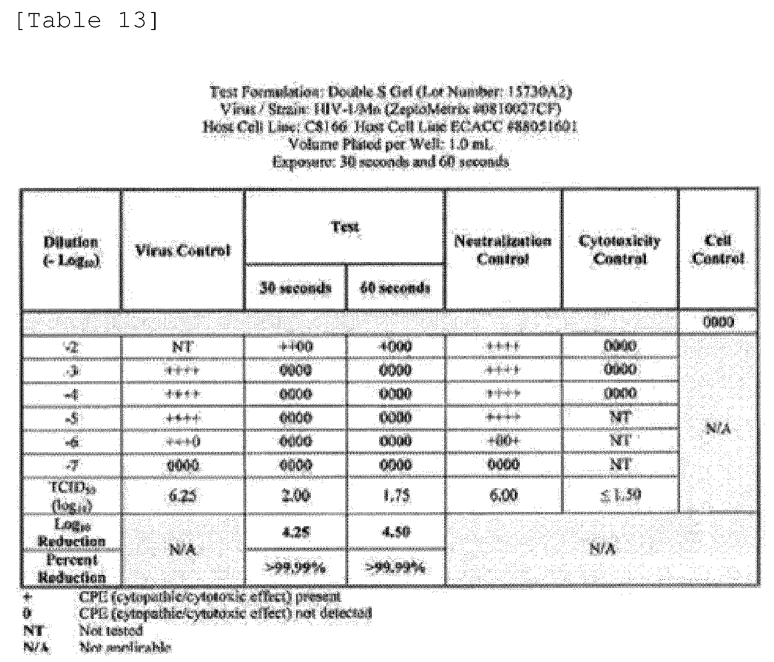

The deodorant cosmetic composition and the method of preparing the same according to the present invention are described with reference to the accompanying drawings. Further, the present invention is illustratively explained by way of the most favorable embodiments, but the invention is not limited to these embodiments.

Therefore, the present invention is not limited to the specific preferred embodiments described above. In addition, it will be understood by those skilled in the art that various changes and modifications may be made without departing from the spirit and scope of the invention as defined by the appended claims. However, such changes and modifications are within the scope of the claims.

## Claims

1. A deodorant cosmetic composition, wherein the deodorant cosmetic composition is prepared by mixing 12 to 18 wt% of butylene glycol, 7 to 13 wt% of glycerin, and 1.11 to 5.85 wt% of additives in 65 to 79.89 wt% of purified water (AQUA Inc.), wherein the additives comprise sodium polyacrylate, a catalyst, a carbomer, an inorganic carrier, a metal salt, a silver precursor, citric acid, and hydroxyethylcellulose.

2. The deodorant cosmetic composition according to claim 1, wherein the additives are obtained by mixing 1 to 4 wt% of the sodium polyacrylate, 0.01 to 0.30 wt% of the catalyst, 0.01 to 0.30 wt% of the carbomer, 0.01 to 0.30 wt% of the inorganic carrier, 0.01 to 0.30 wt% of the metal salt, 0.05 to 0.30 wt% of the silver precursor, 0.01 to 0.05 wt% of the citric acid, and 0.01 to 0.30 wt% of the hydroxyethylcellulose.

3. The deodorant cosmetic composition according to claim 2, wherein the catalyst is at least one selected from the group consisting of aqueous ammonia, ammonium bicarbonate, and triethanolamine.

4. The deodorant cosmetic composition according to claim 2, wherein the inorganic carrier is at least one selected from the group consisting of silica, zeolite, and zirconium, which have an average particle diameter of 100 to 150 nm.

5. The deodorant cosmetic composition according to claim 2, wherein the metal salt is at least one selected from the group consisting of metal nitrates, metal phosphates, and metal carbonates.

6. The deodorant cosmetic composition according to claim 2, wherein the silver precursor is at least one selected from the group consisting of silver nitrate, silver nitrite, and silver perchlorate.

7. A method of preparing a deodorant cosmetic composition, comprising:
(a) a step, in which first mixing is performed by mixing 12 to 18 wt% of butylene glycol with 65 to 79.89 wt% of purified water (AQUA Inc.);
(b) a step, in which secondary mixing is performed by mixing the first mixture obtained in step (a) with additives comprising 1 to 4 wt% of sodium polyacrylate, 0.01 to 0.30 wt% of a catalyst, 0.01 to 0.30 wt% of a carbomer, 0.01 to 0.30 wt% of an inorganic carrier, 0.01 to 0.30 wt% of a metal salt, 0.05 to 0.30 wt% of a silver precursor, 0.01 to 0.05 wt% of citric acid, and 0.01 to 0.30 wt% of hydroxyethylcellulose, wherein the additives contain active ingredients having antibacterial, antiviral, and deodorizing activities, and then a reducing agent is added to the secondary mixture to form a bonding layer on a surface of the inorganic carrier, wherein the bonding layer is formed by reacting the metal salt and the silver precursor in the presence of the reducing agent at a temperature range of 35 to 38 °C for 3 to 4 hours; and
(c) a step, in which secondary mixing is performed by mixing the secondary mixture obtained in step (b) with 7 to 13 wt% of glycerin as a dispersion solvent and the mixture is dispersed at a speed of 100 to 500 rpm, and a cleaning gel composition, which has antibacterial, antiviral, and deodorizing activities and is used by application thereof to a specific part of a body, is prepared.

8. The method according to claim 7, wherein the reducing agent is ascorbic acid or sodium borohydride and is used in an amount of 0.1 to 0.5 parts by weight based on 100 parts by weight of the cleaning gel composition.

9. The method according to claim 7, wherein the additives are obtained by mixing 1 to 4 wt% of the sodium polyacrylate, 0.01 to 0.30 wt% of the catalyst, 0.01 to 0.30 wt% of the carbomer, 0.01 to 0.30 wt% of the inorganic carrier, 0.01 to 0.30 wt% of the metal salt, 0.05 to 0.30 wt% of the silver precursor, 0.01 to 0.05 wt% of the citric acid, and 0.01 to 0.30 wt% of the hydroxyethylcellulose.

10. The method according to claim 9, wherein the catalyst is at least one selected from the group consisting of aqueous ammonia, ammonium bicarbonate, and triethanolamine.

11. The method according to claim 9, wherein the inorganic carrier is at least one selected from the group consisting of silica, zeolite, and zirconium, which have an average particle diameter of 100 to 150 nm.

12. The method according to claim 9, wherein the metal salt is at least one selected from the group consisting of metal nitrates, metal phosphates, and metal carbonates.

13. The method according to claim 9, wherein the silver precursor is at least one selected from the group consisting of silver nitrate, silver nitrite, and silver perchlorate.
